(19) Europäisches Patentamt
European Patent Office
Office européen des brevets

(11) Veröffentlichungsnummer : **0 109 606**
**B1**

(12) # EUROPÄISCHE PATENTSCHRIFT

(45) Veröffentlichungstag der Patentschrift : **22.01.86**

(51) Int. Cl.⁴ : **C 07 C 45/49**, C 07 C 47/52

(21) Anmeldenummer : **83111118.2**

(22) Anmeldetag : **08.11.83**

(54) **Verfahren zur Formylierung von Arylhalogeniden.**

(30) Priorität : **18.11.82 DE 3242582**

(43) Veröffentlichungstag der Anmeldung : **30.05.84 Patentblatt 84/22**

(45) Bekanntmachung des Hinweises auf die Patenterteilung : **22.01.86 Patentblatt 86/04**

(84) Benannte Vertragsstaaten : **BE DE FR GB**

(56) Entgegenhaltungen :
**EP-A- 0 064 690**
**DE-B- 1 047 763**
**DE-C- 529 809**
**JOURNAL OF THE AMERICAN CHEMICAL SOCIETY, Band 96, 1974, A. SCHOENBERG et al. "Palladium-Catalyzed Formylation of Aryl, Iteterocyclic, and Vinylic Halides" Seiten 7761-7764**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Fiedler, Paul, Dr.**
**Rybniker Strasse 8**
**D-5000 Koeln 80 (DE)**
Erfinder : **Braden, Rudolf, Dr.**
**Nothauserfeld 1**
**D-5068 Odenthal (DE)**

**EP 0 109 606 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft ein Verfahren zur Formylierung von Arylhalogeniden mit Kohlenmonoxid und Wasserstoff in Gegenwart eines Edelmetallkatalysators, tertiären organischen Stickstoffverbindungen und Phosphanen und/oder Phosphiten als Promotor.

Die Formylierung von Arylbromiden und Aryljodiden ist bekannt (J. Am. Chem. Soc. *96,* 7761 (1974)). Dabei wird in Gegenwart einer tertiären Stickstoffverbindung und 1,45 bis 2,9 Mol-% eines Dibromo-bis(triphenylphosphan)-palladium(II)-Komplexes gearbeitet und Raum-Zeit-Ausbeuten zwischen 2 und 44 g/l · h erzielt, wenn man als Raum das Volumen der flüssigen Reaktionsphase ansieht. Wegen des hohen Aufwandes an teuren Palladium-Katalysatoren und den geringen Raum-Zeit-Ausbeuten hat dieses bekannte Verfahren keine technische Anwendung gefunden.

Es wurde ein Verfahren zur Herstellung von Aldehyden der Formel

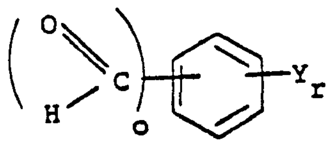

in der

o für eine der Zahlen 1, 2 oder 3 steht,

r für eine der Zahlen 1, 2, 3, 4 oder 5 steht und

Y für Wasserstoff, Fluor, Alkyl, Aryl oder eine der Gruppen

$$-OR,\ -\overset{O}{\overset{\|}{C}}-OR,\ -O-\overset{O}{\overset{\|}{C}}-R,\ -OCF_3,\ -OCCl_3,\ -SR,\ -CClF_2,\ -\overset{O}{\overset{\|}{C}}-CClF_2$$

oder —CN steht, wobei R für Alkyl oder Aryl steht, wobei für den Fall, daß r für eine der Zahlen 2, 3, 4 oder 5 steht, Y jeweils gleich oder verschieden sein kann und wobei für den Fall, daß zwei Y benachbart sind, diese zu einem Ring verbunden sein können, durch Formylierung von Arylhalogeniden der Formel

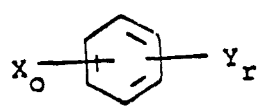

in der

X Chlor, Brom oder Jod bedeutet,

o, r und Y die oben angegebene Bedeutung haben und

wobei für den Fall, daß o für eine der Zahlen 2 oder 3 steht, X jeweils gleich oder verschieden kann, mit Kohlenmonoxid und Wasserstoff im Druckbereich von 20 bis 400 bar, im Temperaturbereich von 80 bis 250 °C, in Gegenwart einer tertiären organischen Stickstoffverbindung der Formel

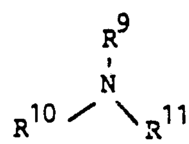

in der $R^9$, $R^{10}$ und $R^{11}$ gleich oder verschieden sind und Alkyl mit 1 bis 20 C-Atomen oder Cycloalkyl mit 5 bis 12 C-Atomen bedeuten, in einem Verhältnis von 1 : 1 bis 5 : 1, bezogen auf das Arylhalogenid, in Gegenwart von Palladium oder einer Palladiumverbindung als Katalysator, in Gegenwart von Phosphanen und/oder Phosphiten und in flüssiger Phase, gefunden, das dadurch gekennzeichnet ist, daß man den Katalysator in einer Menge von 0,01 bis 1 Mol-%, bezogen auf das Arylhalogenid, einsetzt und in Gegenwart von Phosphanen der Formel

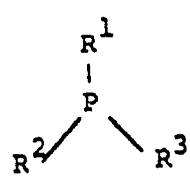

und/oder Phosphiten der Formel

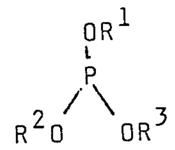

in denen $R^1$, $R^2$ und $R^3$ gleich oder verschieden sind und Alkyl, Cycloalkyl, Aryl oder Aralkyl bedeuten, wobei diese Reste gegebenenfalls durch Hydroxy, Alkoxy, Carbalkoxy, Amino oder Halogen substituiert sein können, in der 10- bis 1 000-fachen molaren Menge des Katalysators arbeitet.

Für das erfindungsgemäße Verfahren seien bevorzugt Phosphane der Formel

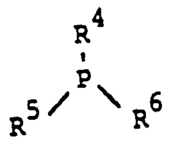

und Phosphite der Formel

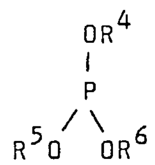

in denen $R^4$, $R^5$ und $R^6$ gleich oder verschieden sind, Alkyl mit 1 bis 20 Kohlenstoffatomen, Cycloalkyl mit 5 bis 12 Kohlenstoffatomen, Aralkylreste mit 7 bis 12 Kohlenstoffatomen oder Arylreste mit 6 bis 18 Kohlenstoffatomen bedeuten, wobei die Reste gegebenenfalls durch Hydroxy, Niederalkoxy, Niedercarboalkoxy, Fluor, Chlor oder Amino der Formel

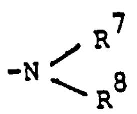

in der $R^7$ und $R^8$ gleich oder verschieden sind und Niederalkyl oder die Gruppe

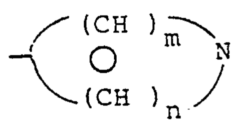

in der die Gesamtzahl der Ringatome 5 oder 6 beträgt und m und n für 0, 1 oder 2 stehen, substituiert sein können, bedeuten, genannt.

Alkyl kann hierbei erfindungsgemäß ein geradkettiger oder verzweigter Kohlenwasserstoffrest mit 1 bis 20, bevorzugt 1 bis 12, Kohlenstoffatomen sein. Im besonderen bevorzugt wird der Niederalkylrest mit 1 bis 6 Kohlenstoffatomen. Beispielsweise seien die folgenden Alkylreste genannt : Methyl, Ethyl, Propyl, Isopropyl, Butyl, Isobutyl, Pentyl, Isopentyl, Hexyl und Isohexyl.

Cycloalkyl kann hierbei erfindungsgemäß ein cyclischer Kohlenwasserstoffrest mit 5 bis 12, bevorzugt 5 bis 7, Kohlenstoffatomen sein. Im besonderen bevorzugt sei der Cyclopentyl-, der Cyclohexyl- und der Cycloheptylrest genannt.

Aralkylreste können hierbei erfindungsgemäß durch Aryl substituierte Alkylreste sein, die im aliphatischen Teil aus einem geradkettigen oder verzweigten Kohlenwasserstoffrest mit 1 bis 6 Kohlenstoffatomen und im aromatischen Teil aus einem Rest der Benzolreihe, vorzugsweise Phenyl, bestehen. Beispielsweise sei als Aralkylrest der Benzylrest genannt.

Aryl kann hierbei erfindungsgemäß ein aromatischer Kohlenwasserstoffrest aus der Benzolreihe mit 6 bis 18, bevorzugt 6 bis 12, Kohlenstoffatomen sein. Beispielsweise seien die folgenden Arylreste genannt : Phenyl, Biphenyl, Naphthyl und Antracyl.

Die Niederalkoxy- und Niedercarbalkoxyreste bestehen hier beim aliphatischen Teil aus einem geradkettigen oder verzweigten Kohlenwasserstoffrest mit 1 bis 6 Kohlenstoffatomen. Beispielsweise seien die folgenden Niederalkoxyreste genannt : Methoxy, Ethoxy, Propoxy, Isopropoxy, Butoxy, Isobutoxy, Pentoxy, Isopentoxy, Hexoxy und Isohexoxy.

Beispielsweise seien die folgenden Niedercarbalkoxyreste genannt : Carbmethoxy, Carbethoxy, Carbisopropoxy und Carbpropoxy.

Halogen kann hierbei erfindungsgemäß Fluor oder Chlor, bevorzugt Fluor, sein.

Die Phosphane für das erfindungsgemäße Verfahren sind an sich bekannt (Houben-Weyl, Meth. der Org. Chem. Bd. XII/1).

Sie können beispielsweise durch Umsetzung von $PCl_3$ mit den entsprechenden Grignard-Verbindungen hergestellt werden.

Die Phosphite für das erfindungsgemäße Verfahren sind an sich bekannt (Houben-Weyl, Meth. d. Org. Chemie Bd. XII/2).

Sie können beispielsweise durch Umsetzung von Alkoholen mit Phosphortrichlorid in Gegenwart eines säurebindenden Mittels wie Triethylamin hergestellt werden.

Beispielsweise seien die folgenden Phosphane und Phosphite genannt: Triphenylphosphan, Triphenylphosphit, Diethylphenylphosphan, Diethylphenylphosphit, Tritolylphosphan, Tritolylphosphit, Trinaphthylphosphan, Trinaphthylphosphit, Diphenylmethylphosphan, Diphenylmethylphosphit, Diphenylbutylphosphan, Diphenylbutylphosphit, Tris-(p-carbmethoxyphenyl)-phosphan, Tris-(p-carbmethoxyphenyl)-phosphit, Tris-(p-cyanophenyl)-phosphan, Tris-(p-cyanophenyl)-phosphit, Triethylphosphit, Tributylphosphan, Tributylphosphit, $P[CH_2CH_2CH_2N(CH_3)_2]_3$, $P[OCH_2CH_2CH_2N(CH_3)_2]_3$, $P[CH_2CH_2CH_2N(C_2H_5)_2]_3$, $P[OCH_2CH_2CH_2N(C_2H_5)_2]_3$, $P(CH_2CH_2\text{-NH-iso-}C_4H_9)_3$, $P(OCH_2CH_2CH_2\text{-NH-iso-}C_4H_9)_3$, $P[CH_2CH_2CH_2N(\text{iso-}C_4H_9)_2]_3$, $P[OCH_2CH_2CH_2N(\text{iso-}C_4H_9)]_3$, $(n\text{-}C_4H_9)_2PCH_2CH_2N(C_2H_5)_2$, $P[CH_2N(C_2H_5)_2]_3$, $P[OCH_2N(C_2H_5)_2]_3$, $P[C_6H_4N(CH_3)_2]_3$, $P[OC_6H_4N(CH_3)_2]_3$, $P[CH_2CH_2C_6H_4N(C_2H_5)_2]_3$, $P[OCH_2CH_2C_6H_4N(C_2H_5)_2]_3$,

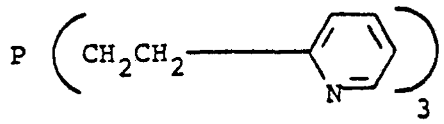

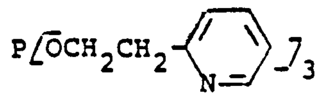

$P[CH_2CH_2CH_2N(\text{tert. }C_4H_9)_2]_3$, $P[OCH_2CH_2CH_2N(\text{tert. }C_4H_9)_2]_3$, $P[CH_2CH_2CH_2N(\text{iso-}C_3H_7)_2]_3$ und $P[OCH_2CH_2CH_2N(\text{iso-}C_3H_7)_2]_3$.

Besonders bevorzugte Phosphane sind Triarylphosphane wie Triphenylphosphan und Tritolylphosphan.

Die Phosphane und Phosphite haben für das erfindungsgemäße Verfahren im wesentlichen eine Promotorwirkung.

Die Phosphane und Phosphite werden in einer Menge angewandt, die der 10- bis 1 000-fachen molaren Katalysatormenge entspricht.

Als Katalysator für das erfindungsgemäße Verfahren wird Palladium oder eine seiner Verbindungen verwendet.

Als Verbindungen des Palladiums seien Komplexe mit den genannten Phosphanen oder Phosphiten genannt. Zweckmäßigerweise verwendet man Palladium-Komplexe der Phosphane oder Phosphite, die auch als Promotoren verwendet werden. Es ist selbstverständlich aber auch möglich, andere Phosphane oder Phosphite als Liganden des Palladiums zu verwenden.

Der Katalysator wird in einer Menge von $10^{-4}$ bis $10^{-2}$ Mol, bezogen auf das Ausgangsprodukt, verwendet.

Die Verwendung von tertiären organischen Stickstoffverbindungen für Formylierungsreaktionen ist an sich bekannt (J. Am. Chem. Soc. *96* 7761 (1974)).

Bevorzugt werden hierbei tertiäre organische Stickstoffverbindungen der Formel

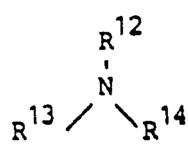

in der $R^{12}$, $R^{13}$ und $R^{14}$ gleich oder verschieden sind und Alkyl mit 1 bis 6 C-Atomen oder Cycloalkyl mit 5 bis 8 C-Atomen bedeuten.

Beispielsweise seien die folgenden tertiären organischen Stickstoffverbindungen genannt: Triethylamin, Tributylamin, Dicyclohexylmethylamin.

In dem erfindungsgemäßen Verfahren verwendet man die tertiären organischen Stickstoffverbindungen in einem Verhältnis von 1 : 1 bis 5 : 1, bezogen auf das Ausgangsprodukt.

Das erfindungsgemäße Verfahren wird im Temperaturbereich von 80 bis 250 °C, bevorzugt im Temperaturbereich von 100 bis 190 °C, durchgeführt.

Das erfindungsgemäße Verfahren wird im Druckbereich von 20 bis 400 bar, bevorzugt im Druckbereich von 70 bis 300 bar, durchgeführt.

Bei dem erfindungsgemäßen Verfahren werden Kohlenmonoxid und Wasserstoff in der Regel mindestens im stöchiometrischen Verhältnis, bevorzugt jedoch im Überschuß, z. B. bis zu 1 000 Mol-%, angewandt. Das Gemisch aus Kohlenmonoxid und Wasserstoff enthält Kohlenmonoxid und Wasserstoff

in der Regel im Volumenverhältnis von 1 : 4 bis 4 : 1, besonders bevorzugt im Verhältnis von 2 : 1 bis 1 : 2.

Die tertiäre, organische Stickstoffverbindung wird vorzugsweise in stöchiometrischer Menge, bezogen auf das Arylhalogenid eingesetzt.

Das erfindungsgemäße Verfahren wird in flüssiger Phase durchgeführt. Das flüssige Reaktionsmedium kann entweder ein Gemisch von an sich vorhandenen Flüssigkeiten (d. h. also Einsatz- und Ausgangsprodukten) sein oder gegebenenfalls auch aus einem zugesetzten Lösungsmittel bestehen, das unter den Reaktionsbedingungen inert ist.

Wenn das erfindungsgemäße Verfahren in Gegenwart eines Lösungsmittels durchgeführt wird, verwendet man hierfür organische Lösungsmittel, die sich unter den Reaktionsbedingungen nicht verändern. Beispielsweise seien hier Alkyl- und Alkoxy-substituierte Benzole wie Toluol, Xylole und Anisol, hochsiedende Ester, wie Adipinsäuredimethylester, Ester und Ether von Polyolen, wie Tetraethylenglykoldimethylether, cyclische Ether, wie Tetrahydrofuran und Dioxan, oder Kohlenwasserstoffe, wie Cyclohexan und Heptan, genannt. Als bevorzugte Lösungsmittel werden Cyclohexan und Toluol eingesetzt.

Das Lösungsmittel wird erfindungsgemäß im Verhältnis 20 : 1 bis 1 : 10, bezogen auf das Ausgangsprodukt, angewandt. Bevorzugt ist ein Verhältnis von 5 : 1 bis 1 : 3.

Nach dem erfindungsgemäßen, Verfahren werden gegebenenfalls substituierte Jod-, Brom- und/oder Chlorarylverbindungen formyliert.

Die gegebenenfalls substituierten Jod-, Brom- und/oder Chlorarylverbindungen sind an sich bekannt (Houben-Weyl, Meth. d. Org. Chemie, Bd. V/4) und können beispielsweise durch Umsetzung des Aromaten mit den entsprechenden elementaren Halogen (Jod, Brom oder Chlor) hergestellt werden.

Für das erfindungsgemäße Verfahren werden gegebenenfalls substituierte Jod-, Brom- und/oder Chlorarylaromaten der Formel

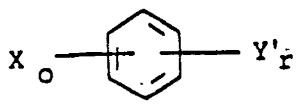

bevorzugt, in der

X, o und r die oben genannte Bedeutung haben und

Y' für Wasserstoff, Fluor, Alkyl mit 1 bis 20 Kohlenstoffatomen, Phenyl oder eine der Gruppen —OR', $—OCF_3$, $—OCCl_3$, —SR', $—CClF_2$ oder —CN steht, wobei R' für Alkyl mit 1 bis 20 Kohlenstoffatomen oder Phenyl steht, wobei für den Fall, daß r für eine der Zahlen 2, 3, 4 oder 5 steht, Y' jeweils gleich oder verschieden sein kann und wobei für den Fall, daß zwei Y' benachbart sind, diese zu einem Ring verbunden sein können.

Besonders bevorzugt werden Verbindungen der Formel

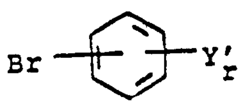

in der Y' und r die oben genannte Bedeutung haben, eingesetzt.

Beispielsweise seien die folgenden, gegebenenfalls substituierten, Jod-, Brom- und/oder Chlorarylaromaten genannt :

Brombenzol, o-, m- und p-Bromtoluol, 2,3-Dimethylbrombenzol, 2,4-Dimethylbrombenzol, 2,5-Dimethylbrombenzol, 2,6-Dimethylbrombenzol, 3,4-Dimethylbrombenzol, 3,5-Dimethylbrombenzol, Brommesitylen, o-, m- und p-Chlorbrombenzol, o-, m- und p-Fluorbrombenzol, 2-Chlor-3-fluorbrombenzol, 2-Chlor-4-fluorbrombenzol, 2-Chlor-5-fluorbrombenzol, 2-Chlor-6-fluorbrombenzol, 3-Chlor-2-fluorbrombenzol, 3-Chlor-4-fluorbrombenzol, 3-Chlor-5-fluorbrombenzol, 3-Chlor-6-fluorbrombenzol, 4-Chlor-2-fluorbrombenzol, 4-Chlor-3-fluorbrombenzol, o-, m- und p-Chloriodbenzol, 2,3-Dichloriodbenzol, 2,4-Dichloriodbenzol, 2,5-Dichloriodbenzol, 2,6-Dichloriodbenzol, 3,4-Dichloriodbenzol, 3,5-Dichloriodbenzol, 2,3,4-Trichloriodbenzol, 2,3,5-Trichloriodbenzol, 2,3,6-Trichloriodbenzol, 2,4,5-Trichloriodbenzol, 2,4,6-Trichloriodbenzol, 3,4,5-Trichloriodbenzol, 2,3,4,5-Tetrachloriodbenzol, 2,3,4,6-Tetrachloriodbenzol, 2,3,5,6-Tetrachloriodbenzol, Pentachloriodbenzol, 2,3-Dichlorbrombenzol, 2,4-Dichlorbrombenzol, 2,5-Dichlorbrombenzol, 2,6-Dichlorbrombenzol, 3,4-Dichlorbrombenzol, 3,5-Dichlorbrombenzol, 2,3,4-Trichlorbrombenzol, 2,3,5-Trichlorbrombenzol, 2,3,6-Trichlorbrombenzol, 2,4,5-Trichlorbrombenzol, 2,4,6-Trichlorbrombenzol, 3,4,5-Trichlorbrombenzol, 2,3,4,5-Tetrachlorbrombenzol, 2,3,5,6-Trichlorbrombenzol, 2,3,4,6-Tetrachlorbrombenzol, Pentachlorbrombenzol, o-, m- und p-Fluoriodbenzol, 2,3-Difluoriodbenzol, 2,4-Difluoriodbenzol, 2,5-Dichloriodbenzol, 2,6-Difluoriodbenzol, 3,4-Difluoriodbenzol, 3,5-Difluoriodbenzol, 2,3,4-Trifluoriodbenzol, 2,3,5-Trifluoriodbenzol, 2,3,6-Trifluoriodbenzol, 2,4,5-Trifluoriodbenzol, 2,4,6-Trifluoriodbenzol, 3,4,5-Trifluoriodbenzol, 2,3,4,5-Tetrafluoriodbenzol, 2,3,5,6-Tetrafluoriodbenzol, 2,3,4,6-Tetrafluoriodbenzol, Pentafluorbrombenzol, 2,3-Difluorbrombenzol, 2,4-Difluorbrombenzol, 2,5-Difluorbrombenzol, 2,6-Difluorbrombenzol, 3,4-Difluorbrombenzol, 3,5-Difluorbrombenzol, 2,3,4-Trifluorbrombenzol, 2,3,5-Trifluorbrombenzol, 2,3,6-Trifluorbrombenzol, 2,4,5-Trifluorbrombenzol, 2,4,6-Trifluorbrombenzol, 2,3,4,5-Tetrafluorbrombenzol, 2,3,4,6-Tetrafluorbrombenzol, 2,3,5,6-Tetrafluorbrombenzol, Pentafluorbrombenzol, o-, m- und p-Trifluormethylbrombenzol, o-, m- und p-Trifluoriodbenzol, o-, m- und p-Trifluormethoxybrombenzol, o-, m- und p-Trifluormethoxyiodbenzol, o-, m- und p-Bromphenolether, o-, m- und p-Brombenzoesäureester, o-, m- und p-Bromphenylester, o-, m- und p-Difluorchlormethylbrombenzol, o-, m- und p-Difluormethoxybrombenzol, o-, m- und p-Brombenzonitril, 1-Bromnaphthalin, 2-Bromnaphthalin, 2-Bromthiophen, 3-Bromthiophen, 2-Brompyridin, 3-Brompyridin, 4-Brompyridin, 2-Brom-3-methylpyridin, 2-Brom-4-methylpyridin, 2-Brom-5-methylpyridin, 2-Brom-6-methylpyridin, 3-Brom-2-methylpyridin, 3-Brom-4-methylpyridin, 3-Brom-5-methylpyridin, 3-Brom-6-methylpyridin, 4-Brom-2-methylpyridin, 4-Brom-3-methylpyridin, 2-Chlor-3-trifluormethylbrombenzol, 2-Chlor-4-trifluormethylbrombenzol, 2-Chlor-5-trifluormethylbrombenzol, 2-Chlor-6-trifluormethylbrombenzol, 3-Chlor-2-trifluormethylbrombenzol, 3-Chlor-4-trifluormethylbrombenzol, 3-Chlor-5-trifluormethylbrombenzol, 3-Chlor-6-trifluormethoxylbrombenzol, 4-Chlor-2-trifluormethylbrombenzol, 4-Chlor-3-trifluormethylbrombenzol, 2-Chlor-3-trifluormethoxybrombenzol, 2-Chlor-4-trifluormethoxybrombenzol, 2-Chlor-5-trifluormethoxybrombenzol, 2-Chlor-6-trifluormethoxybrombenzol, 3-Chlor-2-trifluormethoxybrombenzol, 3-Chlor-4-trifluormethoxybrombenzol, 3-Chlor-5-trifluormethoxybrombenzol, 3-Chlor-6-trifluormethoxybrombenzol, 4-Chlor-2-trifluormethoxybrombenzol, 4-Chlor-3-trifluormethoxybrombenzol, 2-Trichlormethyl-4-trifluormethylbrombenzol, 3-Trichlormethyl-5-trifluormethylbrombenzol, 4-Trichlormethyl-6-trifluormethylbrombenzol, 2-Trichlormethyl-6-trifluormethylbrombenzol, 4,4'-Bis-bromphenoxymethan, 4,4'-Bis-bromphenoxyethan 4,4'-Bis-bromphenoxypropan.

Das erfindungsgemäße Verfahren kann beispielsweise durch die folgende Reaktionsgleichung erläutert werden :

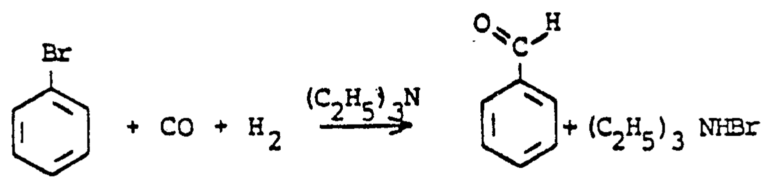

Nach dem erfindungsgemäßen Verfahren werden Aldehyde der Formel

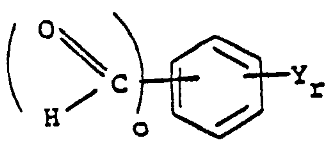

hergestellt, in der Y, r und o die oben genannte Bedeutung haben.

Das erfindungsgemäße Verfahren kann beispielsweise wie folgt durchgeführt werden :

In einem Autoklaven legt man die Jod-, Brom- und/oder Chlorarylverbindung, das tertiäre Amin, das Lösungsmittel, den Edelmetallkatalysator und den Promotor vor. Man erhitzt das Reaktionsgemisch unter Rühren und leitet hierbei ein Kohlenmonoxid-Wasserstoff-Gemisch ein. Die Umsetzung wird solange durchgeführt, bis keine Gasaufnahme mehr erfolgt. Nach dem Abkühlen und Entspannen wird das ausgefallene Salz des tertiären Amins durch Filtration abgetrennt. Das Reaktionsprodukt wird destillativ aufgetrennt. Der Katalysator verbleibt mit den Phosphan oder dem Phosphit im Rückstand und kann in an sich bekannter Weise zurückgewonnen und für weitere Umsetzungen verwendet werden.

Der Destillationsrückstand kann auch direkt als Katalysator-Promotorgemisch für weitere Umsetzungen verwendet werden.

Das erfindungsgemäße Verfahren kann diskontinuierlich aber auch kontinuierlich durchgeführt werden.

Bei dem erfindungsgemäßen Verfahren ist der Katalysatoraufwand stark verringert und die Raum/Zeit-Ausbeute wesentlich erhöht. Überraschenderweise ist es möglich, nach dem erfindungsgemäßen Verfahren auch Chlorarylverbindungen zu formylieren. Diese erhöhte Aktivität des Katalysators ist besonders überraschend, da bekannt ist, daß Phosphane, die z. B. als selektivitätsverbessernde Zusätze bei Hydroformylierungsreaktionen verwendet werden, die Aktivität des Katalysators herabsetzen (New Syntheses with Carbon Monoxide, 93 und 94 (1980)).

Die nach dem erfindungsgemäßen Verfahren hergestellten Aldehyde sind auch Zwischenprodukte für Pflanzenschutzmittel (DE-OS 2 757 066 DE-OS 2 916 358, US 4 191 768 und US 3 992 446).

## Beispiele

In einem 0,3 l Autoklaven aus rostfreiem Stahl legt man 0,20 Mol des Arylhalogenids, 0,22 Mol Triethylamin oder Tributylamin, 80 ml Lösungsmittel und die in Tabelle 1 angegebene Menge Katalysator $PdCl_2(PPh_3)_2$ und Promotor vor. Man erhitzt unter Rühren und Kohlenmonoxid-Wasserstoffdruck auf die angegebene Temperatur und hält den Druck durch regelmäßiges Nachdrücken im angegebenen Bereich, bis keine Gasaufnahme mehr erfolgt. Nach Abkühlen und Entspannen wird das ausgefallene Aminhydrohalogenid durch Filtration abgetrennt und das Filtrat destilliert sowie gaschromatografisch untersucht. Die erhaltenen Werte sind in Tabelle 1 zusammengestellt.

Tabelle 1

| Nr. | Arylhalogenid | Aldehyd | Katalysator [Gew.-%] | Promotor Katalys.-Verhältnis | Promotor | Temperatur °C | Druck bar | Ausbeute % | Raum-Zeit-Ausbeute |
|---|---|---|---|---|---|---|---|---|---|
| 1 | Brombenzol | Benzaldehyd | 0,32 | 100 | $PPh_3$ | 175 | 100 | 88 | 22 |
| 2 | Brombenzol | Benzaldehyd | 2,26 | 100 | $PPh_3$ | 175 | 100 | 95 | 36 |
| 3 | Trifluormethoxy-brombenzol | Trifluormethoxybenzaldehyd | 0,47 | 85 | $PPh_3$ | 175 | 100 | 93 | 30 |
| 4 | 3,4-Dimethyl-brombenzol | 3,4-Dimethyl-benzaldehyd | 1,89 | 100 | $PPh_3$ | 175 | 100 | 82 | 22 |
| 5 | 4-Trifluormethyl-chlorbenzol | 4-Trifluormethylbenzaldehyd | 1,94 | 90 | $PPh_3$ | 175 | 100 | 20 | 5 |
| 6 | Brombenzol | Benzaldehyd | 0,06 | 1000 | $PPh_3$ | 175 | 100 | 57 | 20 |
| 7 | Brombenzol | Benzaldehyd | 0,32 | 100 | (p Cl-)$Ph_3P$ | 175 | 100 | 40 | 25 |
| 8[1)] | Brombenzol | Benzaldehyd | 15,8 | - | - | 125 | 100 | 94 | 0,7 |

1) Beispiel 8 ist ein Vergleichsbeispiel (J. Am. Chem. Soc. *96*, 7761 (1975))
Katalysator in den Beispielen 1 bis 7 : $PdCl_2\ (PPh_3)_3$
Katalysator in dem Beispiel 8 : $PdBr_2\ (PPh_3)_2$
(hierbei steht Ph für Phenyl)

**Patentanspruch**

Verfahren zur Herstellung von Aldehyden der Formel

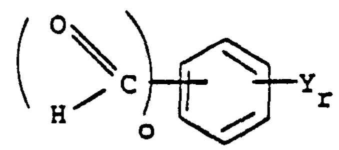

in der
o für eine der Zahlen 1, 2 oder 3 steht,
r für eine der Zahlen 1, 2, 3, 4 oder 5 steht und
Y für Wasserstoff, Fluor, Alkyl, Aryl oder eine der Gruppen

$$-OR,\ -\overset{\overset{O}{\|}}{C}-OR,\ -O-\overset{\overset{O}{\|}}{C}-R,\ -OCF_3,\ -OCCl_3,\ -SR,\ -CClF_2,\ -\overset{\overset{O}{\|}}{C}-CClF_2$$

oder —CN steht, wobei R für Alkyl oder Aryl steht, wobei für den Fall, daß r für eine der Zahlen 2, 3, 4, oder 5 steht, Y jeweils gleich oder verschieden sein kann und wobei für den Fall, daß zwei Y benachbart sind, diese zu einem Ring verbunden sein können, durch Formylierung von Arylhalogeniden der Formel

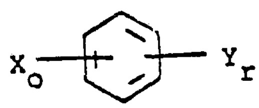

in der
X Chlor, Brom oder Jod bedeutet,
o, r und Y die oben angegebene Bedeutung haben und
wobei für den Fall, daß o für eine der Zahlen 2 oder 3 steht, X jeweils gleich oder verschieden kann, mit Kohlenmonoxid und Wasserstoff im Druckbereich von 20 bis 400 bar, im Temperaturbereich von 80 bis 250 °C, in Gegenwart einer tertiären organischen Stickstoffverbindung der Formel

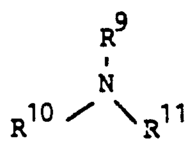

in der $R^9$, $R^{10}$ und $R^{11}$ gleich oder verschieden sind und Alkyl mit 1 bis 20 C-Atomen oder Cycloalkyl mit 5 bis 12 C-Atomen bedeuten, in einem Verhältnis von 1 : 1 bis 5 : 1, bezogen auf das Arylhalogenid, in Gegenwart von Palladium oder einer Palladiumverbindung als Katalysator, in Gegenwart von Phosphanen und/oder Phosphiten und in flüssiger Phase, dadurch gekennzeichnet, daß man den Katalysator in einer Menge von 0,01 bis 1 Mol-%, bezogen auf das Arylhalogenid, einsetzt und in Gegenwart von Phosphanen der Formel

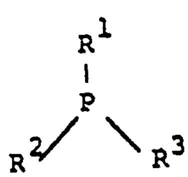

und/oder Phosphiten der Formel

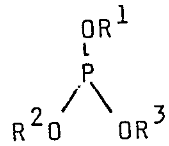

in denen $R^1$, $R^2$ und $R^3$ gleich oder verschieden sind und Alkyl, Cycloalkyl, Aryl oder Aralkyl bedeuten, wobei diese Reste gegebenenfalls durch Hydroxy, Alkoxy, Carboalkoxy, Amino oder Halogen substituiert sein können, in der 10- bis 1 000-fachen molaren Menge des Katalysators arbeitet.

**Claim**

Process for the preparation of aldehydes of the formula

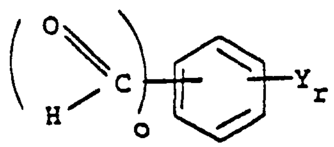

in which
o represents one of the numbers 1, 2 or 3,
r represents one of the numbers 1, 2, 3, 4 or 5 and
Y represents hydrogen, fluorine, alkyl, aryl or one of the groups

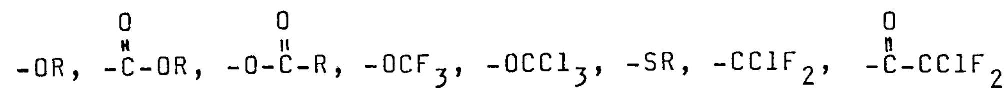

or —CN, wherein R represents alkyl or aryl, wherein in the case where r represents one of the numbers 2, 3, 4 or 5, Y can be identical or different in each case and wherein in the case where two Y's are adjacent they can be bonded to form a ring, by formylating aryl halides of the formula

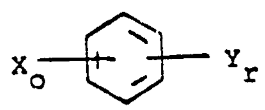

in which
X denotes chlorine, bromine or iodine,
o, r and Y have the meaning given above and
wherein in the case where o represents one of the numbers 2 or 3, X can be identical or different in each case, with carbon monoxide and hydrogen under a pressure in the range 20 to 400 bars, at a temperature in the range 80 to 250 °C, in the presence of a tertiary organic nitrogen compound of the formula

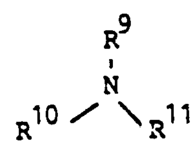

in which $R^9$, $R^{10}$ and $R^{11}$ are identical or different and denote alkyl with 1 to 20 C atoms or cycloalkyl with 5 to 12 C atoms, in a ratio of 1 : 1 to 5 : 1, relative to the aryl halide, in the presence of palladium or a palladium compound as the catalyst, in the presence of phosphanes and/or phosphites and in the liquid phase characterised in that the catalyst is used in a quantity of 0.01 to 1 mol %, relative to the aryl halide, and the reaction is carried out in the presence of phosphanes of the formula

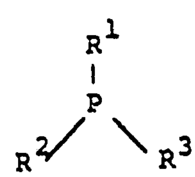

and/or phosphites of the formula

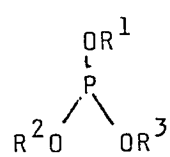

in which $R^1$, $R^2$ and $R^3$ are identical or different and denote alkyl, cycloalkyl, aryl or aralkyl, it being possible for these radicals optionally to be substituted by hydroxyl, alkoxy, carboalkoxy, amino or halogen, in 10 to 1,000 times the molar quantity of the catalyst.

**Revendication**

Procédé de production d'aldéhydes de formule

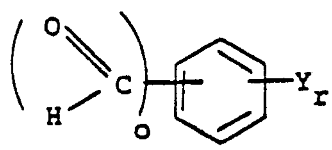

dans laquelle
o représente l'un des nombres 1, 2 ou 3,
r représente l'un des nombres 1, 2, 3, 4 ou 5 et
Y désigne l'hydrogène, le fluor, un groupe alkyle, aryle ou l'un des groupes

$$-OR,\ -\overset{O}{\overset{\|}{C}}-OR,\ -O-\overset{O}{\overset{\|}{C}}-R,\ -OCF_3,\ -OCCl_3,\ -SR,\ -CClF_2,\ -\overset{O}{\overset{\|}{C}}-CClF_2$$

ou —CN, où R désigne un groupe alkyle ou aryle, au cas où r représente l'un des nombres 2, 3, 4 ou 5, les groupes Y peuvent dans chaque cas être égaux ou différents et au cas où deux groupes Y sont adjacents, ils peuvent former conjointement un noyau, par formylation d'halogénures d'aryle de formule

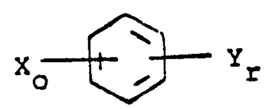

dans laquelle
X représente le chlore, le brome ou l'iode,
o, r et Y ont la définition indiquée ci-dessus et
au cas où o représente l'un des nombres 2 ou 3, les groupes X peuvent être égaux ou différents, avec l'oxyde de carbone et l'hydrogène dans l'intervalle de pression de 20 à 400 bars, dans la plage de température de 80 à 250 °C, en présence d'un composé azoté organique tertiaire de formule

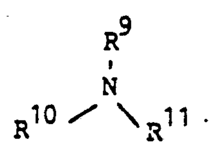

dans laquelle $R^9$, $R^{10}$ et $R^{11}$ sont égaux ou différents et représentent un groupe alkyle ayant 1 à 20 atomes de carbone ou un groupe cycloalkyle ayant 5 à 12 atomes de carbone, dans un rapport de 1 : 1 à 5 : 1, relativement à l'halogénure d'aryle, en présence de palladium ou d'un composé de palladium comme catalyseur, en présence de phosphanes et/ou de phosphites et en phase liquide, caractérisé en ce qu'on utilise le catalyseur en une quantité de 0,01 à 1 mole % par rapport à l'halogénure d'aryle et on opère en présence de phosphanes de formule

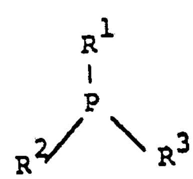

et/ou de phosphites de formule

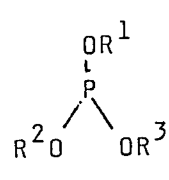

dans lesquelles $R^1$, $R^2$ et $R^3$ sont égaux ou différents et désignent des restes alkyle, cycloalkyle, aryle ou aralkyle, ces restes pouvant éventuellement être substitués par des radicaux hydroxy, alkoxy, carbalkoxy, amino ou halogéno, dans 10 à 1 000 fois la quantité molaire du catalyseur.